# EUROPEAN PATENT APPLICATION

(11) **EP 4 736 866 A1**
(43) Date of publication of application: **06.05.2026**
(21) Application number: 24830963.5
(22) Date of filing: 28.06.2024
(51) Int. Cl.: A61K 38/44, C12N 9/06, A61P 19/06

(54) **STABILIZER FOR URATE OXIDASE AND PEGYLATED CONJUGATE THEREOF, AND PHARMACEUTICAL USE OF STABILIZER**

(30) Priority: 28.06.2023 CN 202310777108
(71) Applicant: Hangzhou Grand Biologic Pharmaceutical Inc., Hangzhou, Zhejiang 310019 (CN); Peg-Bio Biopharm Co., Ltd. (Chongqing), Chongqing 400714 (CN)
(72) Inventor: WANG, Qian, Hangzhou, Zhejiang 310019 (CN); FAN, Kai, Hangzhou, Zhejiang 310019 (CN); WANG, Yu, Hangzhou, Zhejiang 310019 (CN); LIU, Riyong, Hangzhou, Zhejiang 310019 (CN); WANG, Hongying, Hangzhou, Zhejiang 310019 (CN); HU, Chunlan, Hangzhou, Zhejiang 310019 (CN); YAN, Tianwen, Hangzhou, Zhejiang 310019 (CN); HE, Yunfeng, Hangzhou, Zhejiang 310019 (CN); SU, Guowei, Hangzhou, Zhejiang 310019 (CN); FU, Zhicheng, Hangzhou, Zhejiang 310019 (CN); DING, Xupeng, Hangzhou, Zhejiang 310019 (CN); XU, Ying, Hangzhou, Zhejiang 310019 (CN); CHEN, Zeyu, Hangzhou, Zhejiang 310019 (CN); ZHANG, Hui, Hangzhou, Zhejiang 310019 (CN); ZHOU, Yatong, Hangzhou, Zhejiang 310019 (CN)
(74) Representative: Haseltine Lake Kempner LLP
(86) International application number: PCT/CN2024/102323
(87) International publication number: WO 2025/002334

(57) **Abstract**

Provided is a method for improving the stability of urate oxidase. The inventors have discovered that combining an active ingredient urate oxidase or chemically modified urate oxidase with a stabilizer xanthine through a non-covalent bond can significantly improve the in vivo and in vitro stability of urate oxidase and chemically modified urate oxidase in the form of a tetramer, thereby effectively improving the stability of urate oxidase.

## Description

### FIELD

The present disclosure relates to the field of biology, and more particularly, to a urate oxidase, a stabilizer for PEGylated conjugate thereof, and pharmaceutical use thereof.

### BACKGROUND

Gout is a disease caused by a long-term disorder of purine metabolism or a decrease in uric acid excretion. Its clinical feature is hyperuricemia. Due to the poor solubility of urate, crystals deposit and accumulate subcutaneously and in joints and kidney to form tophi, leading to recurrent acute arthritis and involving the kidneys to cause uric acid stones and interstitial nephritis. In human body, purine is converted into end-product uric acid through enzyme action. Under normal conditions, the uric acid content is 149 to 416 mmol/L in male blood and 89 to 357 mol/L in female blood. The amount of uric acid in the body is about 1,200 mg, and the amount of production and excretion is about 600 mg/day, which is in the equilibrium state. However, hyperuricemia may be caused by excessive intake of uric acid or dysfunction of the excretion mechanism, resulting in the accumulation of uric acid above 70 mg/L in the blood. When sodium urate reaches saturation in the blood or synovial fluid and crystallizes, or when hyperuricemia lasts for a long time and crystallizes around joints and soft tissues, it can lead to acute gouty arthritis or chronic gouty arthritis and joint deformities. The deposition of urate in the renal tubules and interstitium may induce inflammation that leads to chronic urate nephropathy. In patients with severe hyperuricemia (such as patients with malignant tumors such as leukemia and lymphoma), massive uric acid deposition in a short period of time causes urinary tract obstruction and acute renal failure, also known as uric acid nephropathy.

The cause of hyperuricemia is related to the mutation and inactivation of the uricase gene during human evolution, so humans cannot synthesize active uricase by themselves. The active uricase in non-human primates or other mammals can convert uric acid produced by purine metabolism into more soluble allantoin to improve the excretion efficiency of the kidney (Wortmann R L, Kelley W N. Kelley's textbook of rheumatology (6th). 2001: 1339-1376). Hyperuricemia may be caused by excessive production of uric acid or insufficient excretion of uric acid (Hershfield, Seegmiller, 1976; Roessler, 1995). In recent years, people's quality of life has improved and their living and eating habits have changed, and they consume more high-protein and high-purine foods. In addition, some drugs used during chemotherapy for tumors and drugs such as azathioprine used to prevent organ transplant rejection can cause significant hyperuricemia and may also cause severe gout or renal damage. Mild hyperuricemia before the onset of clinical symptoms can be controlled by restricting diet; however, once clinical symptoms appear, appropriate drug treatment is required. Conventional clinical treatments currently include: analgesic and anti-inflammatory drugs, such as colchicine, ibuprofen, naproxen, etc., which are mainly used to control the symptoms of acute attacks of gouty arthritis, eliminating local pain, swelling and inflammation in joints; uricosuric agents that promote the excretion of uric acid (ineffective if the kidney function is reduced), such as probenicid, sulfinpyrazone, benzbromarone, etc.; drugs that inhibit the synthesis of uric acid, such as allopurinol, which can inhibit xanthine oxidase from converting hypoxanthine or xanthine into uric acid, and instead slowly oxidize it to produce isoxanthine that is easily soluble in water. However, allopurinol therapy may cause hypersensitivity syndromes such as acute renal and liver failure, skin damage, and is unlikely to be effective in patients with severe chronic hyperuricemia who have already developed tophi.

Uricase (EC 1.7.3.3) is widely present in microorganisms (Bacillus fastidious, Candida monocytogenes, Aspergillus flavus), plants (soybeans, chickpeas), animals (pigs, cattle, dogs, baboons) (Suzuki K, Sakasegawa S, Misaki H, Sugiyama M. J Biosci Bioeng. 2004. 98: 153-158). In the presence of oxygen, it can catalyze uric acid to oxidize allantoin to release carbon dioxide (Retailleau P, Colloc'h, Denis V, Francoise B. Acta Cryst D.2004.60: 453-462.). Active uricase is a tetrameric protein composed of identical subunits, each of which has a molecular weight of about 34 kD and is composed of 301 to 304 amino acids. The pH at the highest enzyme activity of uricase in each solution is 8.0 (Bayol A et al. Biophys Chem.1995.54: 229-235.).

In early studies, microorganisms were used to obtain non-recombinant natural uricase. Uric acid or its structural analogs such as adenine, guanine, xanthine, and hypoxanthine were reported as culture medium inducers to increase the expression of urate oxidase (Liu Jianguo et al., Culture conditions for uricase formation of Candida utilis, Wei Sheng Wu Xue Bao, 29(1):45-50, 1989). The results showed that the enzyme activity was the highest when uric acid was used as an inducer, and xanthine and guanine also had an inducing effect. In this induction mechanism, substrate analogs such as purine enter cells and are converted into uric acid under the action of corresponding enzymes, to induce cells to form urate oxidase (Machida, Y.: Agric. Boil. Chem, 44(12), 1980).

Uricase prepared from Aspergillus flavus has been used to treat hyperuricemia caused by leukemia-related chemotherapy for more than 20 years (London and Hudson, 1957; Kissel et al., 1968; Masera et al., 1982; Duke University, CN101280293A). Uricase has better therapeutic effect than allopurinol. For the treatment of gout, infusion of uricase can block acute attacks of hyperuricemia and reduce the size of tophi (Brogard et al., 1978). In 2002, the recombinant Aspergillus flavus uricase produced by Saccharomyces cerevisiae, ELITEK, developed by the French company Sanofi passed FDA certification and was used for the short-term treatment of hyperuricemia caused by tumor chemotherapy. However, when this uricase infusion therapy is used to treat recurrent gout, due to the heterogeneity of the microbial source, the anti-uricase antibodies in the patient's body clear the uricase after repeated infusions, resulting in a rapid decrease in the efficacy of the drug after administration (Donadio et al., 1981; Pui et al., 1997).

Therefore, how to effectively improve the stability of urate oxidase is a key issue that scientific researchers are waiting to solve.

### SUMMARY

The present disclosure aims to solve at least one of the technical problems existing in the prior art at least to some extent.

During the research and development process, the inventors of the present disclosure discovered, unexpectedly and pleasantly, that xanthine can effectively improve the stability of urate oxidase.

To this end, in one aspect of the present disclosure, the present disclosure provides a urate oxidase composition. According to an embodiment of the present disclosure, the urate oxidase composition includes an active ingredient and a stabilizer. The active ingredient includes urate oxidase, and the stabilizer includes xanthine. During the research and development process, the inventors unexpectedly discovered that xanthine can effectively improve the stability of urate oxidase. The urate oxidase composition with xanthine as a stabilizer according to an embodiment of the present disclosure has greatly improved stability.

According to an embodiment of the present disclosure, the urate oxidase composition may further include at least one of the following additional technical features.

According to an embodiment of the present disclosure, a molar ratio of xanthine to urate oxidase tetramer is not less than 0.5:1. According to a specific embodiment of the present disclosure, the lower the uricase oxidase concentration, the more xanthine can be added to the urate oxidase composition system, and the more stable the urate oxidase composition is.

According to an embodiment of the present disclosure, the urate oxidase is extracted natural urate oxidase, expressed recombinant urate oxidase, or modified urate oxidase.

According to a specific embodiment of the present disclosure, the extracted natural urate oxidase is derived from Aspergillus flavus, wild-type Micrococcus, or mammals.

According to a specific embodiment of the present disclosure, the modified urate oxidase is selected from the group consisting of polyethylene glycol-modified urate oxidase, polysaccharide side chain-modified urate oxidase, polyoligopeptide-modified urate oxidase, and a combination thereof.

It should be noted that xanthine can greatly reduce the immunogenicity of enzymes from different sources under the premise of maximizing enzyme activity, which further improves the in vivo stability of urate oxidase.

According to an embodiment of the present disclosure, the urate oxidase is bound to xanthine through a non-covalent bond. In this way, the in vivo and in vitro stability of urate oxidase and chemically modified urate oxidase in the form of a tetramer can be significantly improved.

According to a specific embodiment of the present disclosure, the urate oxidase is bound to xanthine through hydrogen bonding, salt bonding, hydrophobic interaction, or van der Waals force.

According to an embodiment of the present disclosure, a concentration of the urate oxidase in the composition ranges from 1 mg/ml to 12 mg/ml, and a molar ratio of xanthine to urate oxidase tetramer is (0.5 to 200): 1.

According to an embodiment of the present disclosure, a concentration of the urate oxidase in the composition ranges from 1 mg/ml to 12 mg/ml, and a molar ratio of xanthine to urate oxidase tetramer is (1 to 156): 1. Within this range, the stability of urate oxidase is higher.

According to an embodiment of the present disclosure, a concentration of the urate oxidase in the composition is 1 mg/ml, and a molar ratio of xanthine to urate oxidase tetramer is (0.5 to 200):1.

According to an embodiment of the present disclosure, a concentration of the urate oxidase in the composition is 1 mg/ml, and a molar ratio of xanthine to urate oxidase tetramer is (1 to 156):1.

According to an embodiment of the present disclosure, a concentration of the urate oxidase in the composition is 6 mg/ml, and a molar ratio of xanthine to urate oxidase tetramer is (0.5 to 34):1.

According to an embodiment of the present disclosure, a concentration of the urate oxidase in the composition is 6 mg/ml, and a molar ratio of xanthine to urate oxidase tetramer is (0.5 to 33.3):1.

According to an embodiment of the present disclosure, a concentration of the urate oxidase in the composition is 6 mg/ml, and a molar ratio of xanthine to urate oxidase tetramer is (1 to 26):1.

According to an embodiment of the present disclosure, a concentration of the urate oxidase in the composition is 8 mg/ml, and a molar ratio of xanthine to urate oxidase tetramer is (0.5 to 25):1.

According to an embodiment of the present disclosure, a concentration of the urate oxidase in the composition is 8 mg/ml, and a molar ratio of xanthine to urate oxidase tetramer is (1 to 20):1.

According to an embodiment of the present disclosure, a concentration of the urate oxidase in the composition is 8 mg/ml, and a molar ratio of xanthine to urate oxidase tetramer is (1 to 19.5):1.

According to an embodiment of the present disclosure, a concentration of the urate oxidase in the composition is 12 mg/ml, and a molar ratio of xanthine to urate oxidase tetramer is (0.5 to 17):1.

According to an embodiment of the present disclosure, a concentration of the urate oxidase in the composition is 12 mg/ml, and a molar ratio of xanthine to urate oxidase tetramer is (0.5 to 16.7):1.

According to an embodiment of the present disclosure, a concentration of the urate oxidase in the composition is 12 mg/ml, and a molar ratio of xanthine to urate oxidase tetramer is (1 to 13):1.

It should be noted that the urate oxidase or the polyethylene glycol-modified urate oxidase involved in the present disclosure exists in the form of a tetramer. The molar ratio of xanthine to urate oxidase mentioned herein refers to the molar ratio of xanthine to urate oxidase tetramer. In addition, when the molar ratio of xanthine to urate oxidase is involved, the urate oxidase may be urate oxidase tetramer or polyethylene glycol-modified urate oxidase tetramer.

According to an embodiment of the present disclosure, the urate oxidase composition further includes a pharmaceutically acceptable excipient or carrier. The excipient or carrier is selected from the group consisting of hydroxypropyl methylcellulose, hydroxyethyl cellulose, hydroxypropyl cellulose, mannitol, sucrose, fructose, methyl cellulose, vinyl acetate copolymer, chitosan, sodium alginate, xanthan gum, sodium carboxymethyl cellulose, polyethylene oxide, methacrylic acid copolymer, maleic anhydride-methyl vinyl ether copolymer, carbomer, polyvinyl pyrrolidone, ethyl cellulose, cellulose acetate, methacrylate, polyoxyethylene, polyvinyl alcohol, glyceryl behenate, glycerol monostearate, cellulose acetate, cellulose acetate phthalate, ethyl cellulose, PLA, PLGA, polyethylene glycol, and a combination thereof.

According to an embodiment of the present disclosure, the urate oxidase composition is an injection. Therefore, the urate oxidase composition can be distributed in the body more quickly, thereby achieving a better therapeutic effect.

In another aspect of the present disclosure, the present disclosure provides use of xanthine in improving the stability of urate oxidase. The inventors have discovered that xanthine can significantly improve the in vivo and in vitro stability of urate oxidase and chemically modified urate oxidase in the form of a tetramer by specifically binding to urate oxidase and chemically modified urate oxidase through a non-covalent bond. According to an embodiment of the present disclosure, xanthine is introduced during the preparation of urate oxidase and modified urate oxidase, and xanthine combines with urate oxidase to effectively improve the stability of urate oxidase.

In yet another aspect of the present disclosure, the present disclosure provides a method for improving the stability of urate oxidase. According to an embodiment of the present disclosure, the method includes contacting the urate oxidase with xanthine. According to the method of the embodiment of the present disclosure, urate oxidase can be specifically bound to xanthine, thereby effectively improving the stability of urate oxidase.

In a further aspect of the present disclosure, the present disclosure provides a method for preparing urate oxidase. According to an embodiment of the present disclosure, the method includes contacting the urate oxidase with xanthine. According to the method of the embodiment of the present disclosure, urate oxidase can be specifically bound to xanthine, thereby effectively improving the stability of urate oxidase.

According to an embodiment of the present disclosure, the urate oxidase is extracted natural urate oxidase, expressed recombinant urate oxidase, or modified urate oxidase.

According to a specific embodiment of the present disclosure, the extracted natural urate oxidase is derived from Aspergillus flavus, wild-type Micrococcus, or mammals.

The sequence information of urate oxidase derived from Aspergillus flavus is as follows:

SAVKAARYGKDNVRVYKVHKDEKTGVQTVYEMTVCVLLEGEIETSYTKA
DNSVIVATDSIKNTIYITAKQNPVTPPELFGSILGTHFIEKYNHIHAAHVNIVCHRWTRM
DIDGKPHPHSFIRDSEEKRNVQVDVVEGKGIDIKSSLSGLTVLKSTNSQFWGFLRDEY
TTLKETWDRILSTDVDATWQWKNFSGLQEVRSHVPKFDATWATAREVTLKTFAEDNS
ASVQATMYKMAEQILARQQLIETVEYSLPNKHYFEIDLSWHKGLQNTGKNAEVFAPQ
SDPNGLIKCTVGRSSLKSKL (SEQ ID NO: 8).

The sequence information of urate oxidase derived from wild-type Micrococcus is as follows:

MTNIILGKNQYGKAEVRLVKVTRNTCRHEIQDLNVTSQLRGDFEAAHTAG
DNAHVVATDTQKNTVYAFARDGFATTEEFLLRLGKHFTEGFDWVTGGRWAAQQFFW
DRINDHDHAFSRNKSEVRTAVLEISGSEQAIVAGIEGLTVLKSTGSEFHGFPRDKYTTLP
ETEDRILATDVSARWRYNTVEVDFDAVYADVRGLLLKAFAETHSLALQQTMYEMGR
AVIETHPEIDEIKMSLPNKHHFLVDLSPFGQDNPNEVFYAADRPYGLIEATIQREGCRA
DHPIWSN (SEQ ID NO: 9).

According to a specific embodiment of the present disclosure, the modified urate oxidase is selected from the group consisting of polyethylene glycol-modified urate oxidase, polysaccharide side chain-modified urate oxidase, polyoligopeptide-modified urate oxidase, and a combination thereof.

It should be noted that xanthine can greatly reduce the immunogenicity of enzymes from different sources under the premise of maximizing enzyme activity, which further improves the in vivo stability of urate oxidase.

According to an embodiment of the present disclosure, the contact between the urate oxidase and xanthine is performed by: fermenting engineered bacteria carrying a nucleic acid molecule expressing the urate oxidase in a xanthine-containing fermentation broth to obtain the urate oxidase. Therefore, xanthine can more fully bind to urate oxidase and improve the stability of urate oxidase.

According to an embodiment of the present disclosure, an amount of xanthine in the xanthine-containing fermentation broth is not less than 0.1 g/L.

According to an embodiment of the present disclosure, the contact between the urate oxidase and xanthine is performed by: fermenting engineered bacteria carrying a nucleic acid molecule expressing the urate oxidase in a xanthine-containing fermentation broth to obtain the urate oxidase, and performing a polyethylene glycol modification and purification treatment on the urate oxidase to obtain polyethylene glycol-modified urate oxidase. Therefore, the stability of urate oxidase can be improved.

According to an embodiment of the present disclosure, the contact between the urate oxidase and xanthine is performed by: fermenting engineered bacteria carrying a nucleic acid molecule expressing the urate oxidase to obtain the urate oxidase, and performing a polyethylene glycol modification and purification treatment on the urate oxidase to obtain polyethylene glycol-modified urate oxidase. The fermentation system contains or does not contain xanthine. Adding xanthine to the polyethylene glycol modification or purification system means contacting the polyethylene glycol-modified urate oxidase with xanthine. After adding xanthine to PEGylated urate oxidase tetramer, the system exhibits a more stable trend, and the addition of xanthine has no effect on the activity of the PEGylated urate oxidase.

In yet another aspect of the present disclosure, the present disclosure provides a urate oxidase. According to an embodiment of the present disclosure, the urate oxidase is prepared according to the method described above. Therefore, the obtained urate oxidase has high stability.

In yet another aspect of the present disclosure, the present disclosure provides use of the above-mentioned urate oxidase composition or the above-mentioned urate oxidase in the manufacture of a medicament. The medicament is used for preventing or treating a hyperuricemia-related disease.

According to an embodiment of the present disclosure, the hyperuricemia-related disease includes chronic hyperuricemia, gout, kidney disease, hyperuricemia arthritis, kidney stones, gouty nodules, hypertension, diabetes, hypertriglyceridemia, metabolic syndrome, coronary heart disease, atherosclerosis, or hyperuricemia caused by cancer chemotherapy.

In yet another aspect of the present disclosure, the present disclosure provides use of the above-mentioned urate oxidase composition or the above-mentioned urate oxidase in reducing a uric acid level in a biological fluid.

According to an embodiment of the present disclosure, the biological fluid is urine or blood.

It can be understood that the technical effects of the additional technical features of the above-mentioned urate oxidase composition are applicable to the additional technical features of the method for improving the stability of urate oxidase and the method for preparing urate oxidase according to embodiments of the present disclosure, and the additional technical features of the method for improving the stability of urate oxidase and the method for preparing urate oxidase according to embodiments of the present disclosure are not described in detail herein.

In yet another aspect of the present disclosure, the present disclosure provides a medicament. According to an embodiment of the present disclosure, the medicament includes the above-mentioned urate oxidase composition or the above-mentioned urate oxidase and a pharmaceutically acceptable carrier and/or excipient. As mentioned above, xanthine can bind to urate oxidase and improve the stability of urate oxidase. Therefore, the medicament has good stability, and can be stored for a long time and maintain good activity.

In another aspect of the present disclosure, the present disclosure provides use of the above-mentioned urate oxidase composition or the above-mentioned urate oxidase in preventing or treating a hyperuricemia-related disease.

In yet another aspect of the present disclosure, the present disclosure provides a method for preventing or treating a hyperuricemia-related disease. According to an embodiment of the present disclosure, the method includes administering to a subject a therapeutically effective amount of the above-mentioned urate oxidase composition, the above-mentioned urate oxidase, or the above-mentioned medicament.

According to an embodiment of the present disclosure, the hyperuricemia-related disease includes chronic hyperuricemia, gout, kidney disease, hyperuricemia arthritis, kidney stones, gouty nodules, hypertension, diabetes, hypertriglyceridemia, metabolic syndrome, coronary heart disease, atherosclerosis, or hyperuricemia caused by cancer chemotherapy.

Additional aspects and advantages of the present disclosure will be provided in part in the following description, or will become apparent in part from the following description, or can be learned from practicing of the present disclosure.

### BRIEF DESCRIPTION OF THE DRAWINGS

The foregoing and/or additional aspects and advantages of the present disclosure will become apparent and readily appreciated from the following description of the embodiments in conjunction with the accompanying drawings of which:
FIG. 1 shows an RP-HPLC graph of a urate oxidase with xanthine introduced during fermentation according to an embodiment of the present disclosure;
FIG. 2 shows an SEC-HPLC graph of a urate oxidase with xanthine introduced during fermentation according to an embodiment of the present disclosure;
FIG. 3 shows a chromatogram of a urate oxidase without adding xanthine during purification according to an embodiment of the present disclosure;
FIG. 4 shows a chromatogram of a urate oxidase with xanthine added during purification according to an embodiment of the present disclosure;
FIG. 5 shows a curve graph of changes in blood concentration according to an embodiment of the present disclosure; and
FIG. 6 shows a graph of changes in uric acid levels in serum according to an embodiment of the present disclosure.

### DETAILED DESCRIPTION

The embodiments of the present disclosure are described in detail below, and examples of the embodiments are illustrated in the accompanying drawings, throughout which same or similar reference numerals represent same or similar components or components with same or similar functions. The embodiments described below with reference to the accompanying drawings are illustrative and are intended to explain the present disclosure, but should not be understood as a limitation of the present disclosure.

Furthermore, the terms "first" and "second" are used for descriptive purposes only and are not to be construed as indicating or implying relative importance or implicitly indicating the number of technical features indicated. Thus, a feature defined as "first" or "second" may explicitly or implicitly include at least one such feature. In the description of the present disclosure, "plurality" means at least two, e.g., two, three, etc., unless specifically limited otherwise.

As used herein, the terms "urate oxidase" and "uricase" are interchangeable, and they both refer to a class of enzymes described in the present disclosure that can catalyze the oxidation of uric acid to produce allantoin and hydrogen peroxide. The terms "urate oxidase analogue", "uricase analogue", and "uricase derivative" are interchangeable, and they all refer to those in which a structural modification, such as substitution, deletion, or addition of some amino acids, is subjected to the protein structural sequence of urate oxidase under the premise that the activity of urate oxidase for specifically catalyzing the conversion of uric acid into allantoin and hydrogen peroxide is maintained, or those in which xanthine according to an embodiment of the present disclosure is added to the urate oxidase in a preparation process, thereby achieving the advantages of the present disclosure, including but not limited to increasing protein stability.

Urate oxidase is not particularly limited, and may be urate oxidase and urate oxidase analogues derived from any source. Representative examples include, but are not limited to, mammalian sources, microorganisms, plants, etc.

In another preferred embodiment, the urate oxidase and urate oxidase analogues thereof are derived from mammals, and preferably has an amino acid sequence set forth in SEQ ID NO: 1, SEQ ID NO: 2, SEQ ID NO: 3, or SEQ ID NO: 4, more preferably SEQ ID NO: 1.

TYKKNDEVEFVRTGYGKDMIKVLHIQRDGKYHSIKEVATTVQLTLSSKKD
YLHGDNSDVIPTDTIKNTVNVLAKFKGIKSIETFAVTICEHFLSSFKHVIRAQVYVEEVP
WKRFEKNGVKHVHAFIYTPTGTHFCEVEQIRNGPPVIHSGIKDLKVLKTTQSGFEGFI
KDQFTTLPEVKDRCFATQVYCKWRYHQGRDVDFEATWDTVRSIVLQKFAGPYDKGE
YSPSVQKTLYDIQVLTLGQVPEIEDMEISLPNIHYLNIDMSKMGLINKEEVLLPLDNPY
GKITGTVKRKLSSRL (SEQ ID NO: 1).

MAHYRNDYKKNDEVEFVRTGYGKDMIKVLHIQRDGKYHSIKEVATSVQL
TLSSKKDYLHGDNSDVIPTDTIKNTVNVLAKFKGIKSIETFAVTICEHFLSSFKHVIRAQ
VYVEEVPWKRFEKNGVKHVHAFIYTPTGTHFCEVEQIRNGPPVIHSGIKDLKVLKTTQ
SGFEGFIKDQFTTLPEVKDRCFATQVYCKWRYHQGRDVDFEATWDTVRSIVLQKFAG
PYDKGEYSPSVQKTLYDIQVLTLGQVPEIEDMEISLPNIHYLNIDMSKMGLINKEEVLL
PLDNPYGRITGTVKRKLTSRL (SEQ ID NO: 2).

MYKNDEVEFVRTGYGKDMVKVLHIQRDGKYHSIKEVATSVQLTLSSKKD
YVYGDNSDIIPTDTIKNTVHVLAKFKGIKSIETFAMNICEHFLSSFNHVIRAQVYVEEV
PWKRFEKNGVKHVHAFIHNPTGTHFCEVEQMRSGPPVIHSGIKDLKVLKTTQSGFEG
FIKDQFTTLPEVKDRCFATKVYCKWRYHQGRDVDFEATWDTVRDIVLEKFAGPYDK
GEYSPSVQKTLYDIQVHSLSRVPEMEDMEISLPNIHYFNIDMSKMGLINKEEVLLPLDN
PYGKITGTVKRKLSSRL (SEQ ID NO: 3).

MAHYHNDYKKNDEVEFVRTGYGKDMVKVLHIQRDGKYHSIKEVATSVQL

TLSSKKDYVYGDNSDIIPTDTIKNTVHVLAKFKGIKSIETFAMNICEHFLSSFNHVIRAQ
VYVEEVPWKRFEKNGVKHVHAFIHNPTGTHFCEVEQMRSGPPVIHSGIKDLKVLKTT
QSGFEGFIKDQFTTLPEVKDRCFATKVYCKWRYHQGRDVDFEATWDTVRDIVLEKFA
GPYDKGEYSPSVQKTLYDIQVHSLSRVPEMEDMEISLPNIHYFNIDMSKMGLINKEEV
LLPLDNPYGRITGTAKRKLASKL (SEQ ID NO: 4).

MAHYHNDYQKNDEVEFVRTGYGKDMVKVLHIQRDGKYHSIKEVATSVQL
TLNSRREYLHGDNSDIIPTDTIKNTVQVLAKFKGIKSIETFAMNICEHFLSSFNHVIRVQ
VYVEEVPWKRFEKNGVKHVHAFIHTPTGTHFCEVEQLRSGPPVIHSGIKDLKVLKTT
QSGFEGFLKDQFTTLPEVKDRCFATQVYCKWRYHQGRDVDFEATWEAVRGIVLKKFA
GPYDKGEYSPSVQKTLYDIQVLSLSQLPEIEDMEISLPNIHYFNIDMSKMGLINKEEVL
LPLDNPYGRITGTVKRKLTSRL (SEQ ID NO: 5).

MAHYHNDYKKNDEVEFVRTGYGKDMVKVLHIQRDGKYHSIKEVATSVQL
TLSSKKDYLHGDNSDIIPTDTIKNTVHALAKFKGIKSIEAFAVNICQHFLSSFNHVIRTQ
VYVEEIPWKRLEKNGVKHVHAFIHTPTGTHFCEVEQLRSGPPVIHSGIKDLKVLKTTQ
SGFEGFIKDQFTTLPEVKDRCFAAQVYCKWRYHQCRDVDFEATWDTIRDVVLEKFAG
PYDKGEYSPSVQKTLYDIQVVSLSQVPEIDDMEISLPNIHYFNIDMSKMGLINKEEVLL
PLDNPYGKITGTVKRKLSSRL (SEQ ID NO: 6).

MADYHNNYKKNDELEFVRTGYGKDMVKVLHIQRDGKYHSIKEVATSVQL
TLSSKKDYLHGDNSDIIPTDTIKNTVHVLAKFKGIKSIEAFGVNICEYFLSSFNHVIRAQ
VYVEEIPWKRLEKNGVKHVHAFIHTPTGTHFCEVEQLRSGPPVIHSGIKDLKVLKTTQ
SGFEGFIKDQFTTLPEVKDRCFATQVYCKWRYHQCRDVDFEATWGTIRDLVLEKFAG
PYDKGEYSPSVQKTLYDIQVLSLSRVPEIEDMEISLPNIHYFNIDMSKMGLINKEEVLLP
LDNPYGKITGTVKRKLSSRL (SEQ ID NO: 7).

The amino acid sequence set forth in SEQ ID NO: 1 is an amino acid sequence of a chimeric urate oxidase (pig-baboon) derived from pig and baboon; the amino acid sequence set forth in SEQ ID NO: 2 is an amino acid sequence of pig-derived urate oxidase; the amino acid sequence set forth in SEQ ID NO: 3 is an amino acid sequence of a chimeric urate oxidase (canine-baboon) derived from canine and baboon; the amino acid sequence set forth in SEQ ID NO: 4 is an amino acid sequence of canine-derived urate oxidase; the amino acid sequence set forth in SEQ ID NO: 5 is an amino acid sequence of bovine-derived urate oxidase; the amino acid sequence set forth in SEQ ID NO: 6 is an amino acid sequence of monkey-derived urate oxidase; and the amino acid sequence set forth in SEQ ID NO: 7 is an amino acid sequence of baboon-derived urate oxidase.

The urate oxidase having the amino acid sequence set forth in any one of SEQ ID NOs: 1 to 7, or a polypeptide having at least 70%, at least 75%, at least 80%, at least 85%, at least 90%, at least 95%, or at least 99% identity with any one of SEQ ID NOs: 1 to 7, or a polypeptide having one or more amino acid substitutions, deletions, and/or additions and having structural homology with any one of SEQ ID NOs: 1 to, are all within the scope of the urate oxidase of the present disclosure.

The urate oxidase derived from different species according to the present disclosure can be obtained through various manners, including but not limited to natural extraction, chemical synthesis, genetic engineering recombinant expression, etc.

In another preferred embodiment, urate oxidase is obtained by expressing the recombinant coding sequence of the urate oxidase protein sequence (SEQ ID NO: 1) in a host cell by using recombinant technology.

In another preferred embodiment, urate oxidase is prepared and obtained in a manner that E. coli or yeast is used as a host to construct a recombinant expression strain, and E. coli is more preferably used as host bacteria for recombinant expression.

S1 (from Aspergillus flavus) urate oxidase sequence information:

SAVKAARYGKDNVRVYKVHKDEKTGVQTVYEMTVCVLLEGEIETSYTKA
DNSVIVATDSIKNTIYITAKQNPVTPPELFGSILGTHFIEKYNHIHAAHVNIVCHRWTRM
DIDGKPHPHSFIRDSEEKRNVQVDVVEGKGIDIKSSLSGLTVLKSTNSQFWGFLRDEY
TTLKETWDRILSTDVDATWQWKNFSGLQEVRSHVPKFDATWATAREVTLKTFAEDNS
ASVQATMYKMAEQILARQQLIETVEYSLPNKHYFEIDLSWHKGLQNTGKNAEVFAPQ
SDPNGLIKCTVGRSSLKSKL (SEQ ID NO: 8).

S3 (from Micrococcus) urate oxidase sequence information:

MTNIILGKNQYGKAEVRLVKVTRNTCRHEIQDLNVTSQLRGDFEAAHTAG
DNAHVVATDTQKNTVYAFARDGFATTEEFLLRLGKHFTEGFDWVTGGRWAAQQFFW
DRINDHDHAFSRNKSEVRTAVLEISGSEQAIVAGIEGLTVLKSTGSEFHGFPRDKYTTLP
ETEDRILATDVSARWRYNTVEVDFDAVYADVRGLLLKAFAETHSLALQQTMYEMGR

AVIETHPEIDEIKMSLPNKHHFLVDLSPFGQDNPNEVFYAADRPYGLIEATIQREGCRA
DHPIWSN (SEQ ID NO: 9).

As used herein, the PEGylated urate oxidase according to the present disclosure refers to a urate oxidase obtained by covalently modifying urate oxidase with polyethylene glycol. The polyethylene glycol (PEG) refers to a mixture of ethylene oxide condensation and water, represented by the general formula H(OCH₂CH₂)ₙOH. It is a pH-neutral, non-toxic, highly water-soluble hydrophilic polymer with a linear or branched structure.

An object of the present disclosure is to provide a method for preparing a urate oxidase and a chemically modified urate oxidase non-covalently bound to xanthine, which can be introduced in a preparation process of the urate oxidase or chemically modified urate oxidase.

Another object of the present disclosure is to provide a stabilizer for urate oxidase and chemically modified urate oxidase. Further, the stabilizer is xanthine, which can significantly improve the in vivo and in vitro stability of urate oxidase and chemically modified urate oxidase.

Xanthine, widely distributed in human organs and body fluids, is often used as a mild stimulant and bronchodilator and can be used to treat asthma symptoms. The mechanism of action of xanthine drugs is mainly to inhibit phosphodiesterase that can decompose and destroy cAMP, and increase the content of intracellular cAMP, thereby producing a wide range of physiological activities. This type of drug, based on the pharmacological activity, is mainly used for central nervous system excitation, cardiovascular and cerebrovascular dilation, and increasing glomerular filtration rate and primary urine volume to exert diuretic and antiasthmatic effects (Ren Junguo, New Progress in Xanthine Drugs; Foreign Medicine. Synthetic Drugs. Biochemical Drugs. Preparations Volume, 1989). Therefore, as a stabilizer for drug preparation, xanthine has no safety issues itself, and can improve the in vivo and in vitro stability of urate oxidase and PEGylated urate oxidase, thereby improving the safety and efficacy of the drug.

According to the embodiments of the present disclosure, the urate oxidase bound to xanthine of the present disclosure can greatly improve the in vivo stability of urate oxidase while maximizing enzyme activity. Therefore, the polyethylene glycol-modified urate oxidase bound to xanthine and the pharmaceutical composition including the polyethylene glycol-modified urate oxidase of the present disclosure can be administered for the treatment or prevention of hyperuricemia-related diseases.

As used herein, the term "administering" refers to the introduction of a predetermined amount of a substance into a patient in a suitable manner. The polyethylene glycol-modified urate oxidase of the present disclosure can be administered through any common route, as long as it can reach the desired tissue. Various modes of administration are contemplated, including peritoneal, intravenous, intramuscular, subcutaneous, cortical, oral, topical, nasal, pulmonary, and rectal administrations, but the present disclosure is not limited to these exemplified modes of administration. In addition, the pharmaceutical composition of the present disclosure can be administered using a specific device configured to deliver the active ingredient to the target cells.

The administration frequency and dosage of the pharmaceutical composition of the present disclosure can be determined based on a number of related factors, including the type of disease to be treated, the route of administration, the patient's age, gender, and weight, and the severity of the disease, as well as the type of the medicament serving as the active ingredient.

The term "therapeutically effective amount" refers to an amount of a compound that is sufficient to significantly ameliorate certain symptoms associated with a disease or condition, that is, an amount that provides a therapeutic effect for a given condition and dosing regimen. For example, in the treatment of chronic hyperuricemia or gout, drugs or compounds that reduce, prevent, delay, inhibit, or block any symptoms of a disease or disorder should be therapeutically effective. A therapeutically effective amount of the drug or compound is not necessarily to cure the disease or condition, but will provide treatment for the disease or condition such that the onset of the disease or condition of an individual is delayed, blocked, or prevented, or the symptoms of the disease or condition are alleviated, or the duration of the disease or condition is changed, or, for example, the disease or illness becomes less serious, or recovery is accelerated.

The term "subject" refers to any animal, such as a mammal or marsupial. The subjects of the present disclosure include, but are not limited to, humans, non-human primates (e.g., monkeys), mice, pigs, horses, donkeys, cattle, sheep, and any kind of poultry.

The term "treating" or "treatment" is used to refer to obtaining a desired pharmacological and/or physiological effect. Said effect may be prophylactic in terms of complete or partial prevention of the disease or its symptoms, and/or may be therapeutic in terms of partial or complete cure of the disease and/or adverse effects caused by the disease. The "treating" or "treatment" as used herein encompasses the treatment of diseases in mammals, especially human (mainly hyperuricemia-related diseases), including: (a) prevention of diseases in individuals who are prone to diseases but have not yet been diagnosed; (b) inhibition of diseases, such as blocking the progression of the disease; or (c) alleviation of diseases, such as reducing symptoms associated with the disease. The "treating" or "treatment" as used herein encompasses any medication that administers a medicament or compound to an individual to treat, cure, alleviate, ameliorate, reduce or inhibit the individual's disease, including but not limited to administering the polyethylene glycol-modified urate oxidase described herein to the individual in need thereof.

The term "pharmaceutically acceptable carrier" includes any solvent, dispersion medium, coating material, surfactant, antioxidant, preservative (e.g., antibacterial agent, antifungal agent), isotonic agent, salt, drug stabilizer, binder, excipient, dispersant, lubricant, sweetener, flavoring agent, coloring agent, or a combination thereof. These carriers are known to those skilled in the art (e.g., as described in Remington's Pharmaceutical Sciences, 18th Ed. Mack Printing Company, 1990, pp. 1289-1329). Except insofar as any conventional carrier is incompatible with the active ingredient, its use in the therapeutic or pharmaceutical compositions is contemplated.

The term "pharmaceutically acceptable excipient" may include any solvent, solid excipient, diluent, or other liquid excipient suitable for a particular target dosage form. Except to the extent that any conventional excipient is incompatible with the siRNA of the present disclosure, such as any adverse biological effects produced or interactions with any other component of the pharmaceutically acceptable composition in a deleterious manner, their use is also contemplated by the present disclosure.

According to the embodiments of the present disclosure, the urate oxidase or pharmaceutical composition bound to xanthine of the present disclosure can be used in combination with conventional treatment methods and/or therapies, or can be used separately with conventional treatment methods and/or therapies. When the urate oxidase or pharmaceutical composition bound to xanthine of the present disclosure is administered in combination therapy with other drugs, they can be administered to the individual sequentially or simultaneously. Alternatively, the pharmaceutical composition of the present disclosure may include a combination of the urate oxidase bound to xanthine of the present disclosure, a pharmaceutically acceptable carrier or pharmaceutically acceptable excipient, and other therapeutic or preventive drugs known in the art.

It should be understood that, within the scope of the present disclosure, the above-mentioned technical features of the present disclosure and the various technical features specifically described as below (such as in the embodiments) can be combined with each other to form a new or preferred technical solution, which can be understood more clearly by referring to the following examples. Due to limited length, the described examples are for illustrative purposes only and are not intended to limit the present disclosure.

Hereinafter, the embodiments of the present disclosure will be described in further detail, and examples of the embodiments are illustrated in the accompanying drawings. The following embodiments described with reference to the accompanying drawings are illustrative, and are intended to explain the present disclosure, but should not be understood as limitations on the present disclosure.

### Example 1: Preparation of uricase from different sources

Uricase from three different sources (Micrococcus, Aspergillus flavus, and pig-baboon) was prepared. The specific sample information is shown in Table 1.

**[Table 1] Information on urate oxidase and modified urate oxidase from different sources**

| Sample information | Name | Enzyme species | PEG modified? | Sample source |
|---|---|---|---|---|
| S1 | Commercially available product (unmodified) | Aspergillus flavus | No | Commercial sale |
| S2 | PHC | Pig-baboon | No | Homemade |
| S3 | Uricase | Wild-type | No | Homemade |
| | | Micrococcus | | |
| S4 | Commercially available product (modified) | Aspergillus flavus | Yes | Homemade |
| S5 | PU5 | Pig-baboon | Yes | Homemade |
| S6 | Modified uricase | Wild-type | Yes | Homemade |
| | | Micrococcus | | |

### 1. Preparation process of S2 enzyme:

### 1.1 Construction of genes and expression plasmids for uricase expression

According to the usage preference data of E. coli codon, in combination with factors such as codon preference and GC content, the cDNA sequence of uricase protein (referred to as PHC, SEQ ID NO: 1) was designed, and the whole gene was synthesized and named pUC-57-PHC plasmid. Nde I and BamH I were used as the target gene insertion sites, and pET-30a plasmid was used as the expression vector (pET-30a-PHC).

### 1.2 Transformation of expression plasmids into bacterial host cells

The expression vector pET-30a-PHC was introduced into E. coli BL21 (DE3) by the CaCl₂ method. High-expression clones were screened through resistance screening with kanamycin, and the original seed bank strain (E3B) was preserved. These steps were performed in accordance with the commonly used methods in the field of molecular biology.

### 1.3 Preparation of recombinant urate oxidase

The transformed engineered strains were fermented and expressed in a fermentor, under the control conditions: first culturing at 30°C and pH 7.2 to an OD₆₀₀=30 or higher, adding IPTG to 0.5 mmol/L, and inducing for 3 hours or more to allow urate oxidase to accumulate. The cells were collected by centrifugation and then preserved below -15°C.

The frozen bacteria were taken and suspended in a buffer of 25 mmol/L Tris and 5 mmol/L EDTA, at a suspension ratio of 1:10 (W/V). After breaking the bacterial cells with high pressure, the urate oxidase precipitate was collected by centrifugation and washed once with 50 mmol/L NaHCO₃. The enriched uricase precipitate was suspended in a Na₂HCO₃ buffer (100 mmol/L, pH 9.7 to 10.3) at a suspension ratio of 1:50 (W/V), stirred overnight at room temperature, and then centrifuged to collect the supernatant.

Urate oxidase was further purified through several chromatographic steps, exhibiting a purity of greater than 95% by SDS-PAGE and greater than 95% by Superdex 200 column, with no aggregate form. The protein concentration was determined by Lowry method, and the activity of urate oxidase was measured by spectrophotometry, where 1 unit (U) of enzyme activity was defined as the amount of enzyme required to convert 1 µmol of uric acid per minute under the optimal reaction temperature of 37°C and the optimal buffer condition at pH 9.0.

### 2. Preparation process of S5 enzyme:

N-succinimidyl propionate PEG having a molecular weight of 5KD (5K-PEG-SPA) was dissolved with 2 mmol/L HCl to obtain a 200 mmol/L PEG solution, and then added to a carbonate buffer solution with a carbonate concentration of 0.1 to 0.3 mol/L and a pH of 10.0 containing urate oxidase at a molar ratio of 1:55 to 1:95 (urate oxidase: 5K-PEG-SPA), where the molar ratio of urate oxidase to 5K-PEG-SPA was calculated based on the monomeric form of urate oxidase, meaning that the molar ratio of 1:55 to 1:95 referred to a molar ratio of urate oxidase in the monomeric form to 5K-PEG-SPA, thereby allowing a coupling reaction between PEG and urate oxidase. The coupling reaction required stirring at 5 to 30°C for 60 minutes or more, until the PEG coupling degree no longer changed with time. After the reaction was finished, the PEG not participating in the modification and by-products were removed from the reaction by ultrafiltration and/or chromatography. A suitable molecular sieve chromatography medium can be selected to separate and remove the modified by-products. Finally, the 5K PEG-modified PEGylated urate oxidase (referred to as PU5) was obtained through sterile filtration.

### 3. Preparation process of S3 enzyme:

Using E. coli BL21 (DE3) as the host bacteria and PET28a as the expression vector, a nucleic acid sequence (SEQ ID NO: 9) expressing urate oxidase was introduced between the NcoI and XhoI double restriction sites to construct an expression bacterium. Through transformation experiments, the recombinant plasmid containing the S3 enzyme expression gene was introduced into the E. coli BL21 (DE3) host bacteria, and the target strain containing the expression gene was screened in a culture medium containing 50 µg/L kanamycin. The screened strain was cultured overnight in 100 ml LB medium (containing 50 µg/L kanamycin) containing glucose at 30°C, and the OD₆₀₀ was 4.4. The expanded bacterial suspension was inoculated into 5 L of fresh LB medium and cultured at 37°C for 4 hours, and the OD₆₀₀ was about 2.5. After the temperature was lowered to 25°C, IPTG was added to make a final concentration of 0.5 mmol/L to induce the expression of S3 enzyme for about 3.5 hours in total, and then the OD₆₀₀ was about 3.5. After the culture was completed, the bacterial suspension was centrifuged to collect the bacterial cells, thereby obtaining the bacterial cells expressing the S3 enzyme. The fermented bacterial cells were resuspended in a lysis buffer (200 mM NaH₂PO₄-Na₂HPO₄, 50 mM EDTA) at a ratio of 1:10 and crushed by high-pressure homogenization. The supernatant was loaded into Xanthine Agrose filler, eluted and purified with eluent (100 mM Na₂CO₃-NaHCO₃, 0.5 mM uricase), then exchanged with SEC to equilibrium solution (200 mM NaH₂PO₄-Na₂HPO₄), and finally concentrated by ultrafiltration to obtain S3 enzyme.

### 4. Preparation process of S4 enzyme:

Methoxy PEG maleimide (M-PEG-MAL) having a molecular weight of 5KD was dissolved in 1 mmol/L HCl to obtain a 200 mmol/L PEG solution, then added to the S1 enzyme solution at a certain ratio for 1h of modification, and finally concentrated by ultrafiltration to obtain S4 enzyme solution.

### 5. Preparation process of S6 enzyme:

Methoxy PEG maleimide (M-PEG-MAL) having a molecular weight of 5KD was dissolved in 1 mmol/L HCl to obtain a 200 mmol/L PEG solution, added to the S3 enzyme solution at a certain ratio for 1h of modification, and finally concentrated by ultrafiltration to obtain S6 enzyme solution

### Example 2: Stability effect of xanthine on urate oxidase from different sources

### 1. Detection method of high and low molecular proteins of urate oxidase

SEC, 7.8×300 mm chromatographic column or equivalent column was selected. 0.05 mol/L Na₂HPO₄-NaH₂PO₄, 0.3 mol/L NaCl, pH 7.0 was used as the mobile phase. The flow rate of HPLC was set to be 0.5 ml/min, and the detection wavelength to 280 nm. Isocratic elution was performed for 35 min, and the injection volume was 10 µl.

### 2. Detection method of enzyme activity

The method used for determining the activity of urate oxidase and PEGylated urate oxidase was as follows. An ultraviolet (UV) spectrophotometer was used, based on the fact that the substrate uric acid had a maximum ultraviolet absorption wavelength of 293 nm, and within a certain concentration range, the absorption value of uric acid at 293 nm was proportional to its concentration. Therefore, the UV spectrophotometer detection wavelength was 293 nm. The buffer solution was used as a blank control, and the absorption zero point was calibrated. In the quartz cuvette, 2.95 ml of substrate reaction solution was added followed by 50 µl of the test solution, mixed immediately, and the light absorption value at 293 nm was measured. According to C=A/εL (A being the absorbance value of a specific concentration of uric acid at 293 nm, ε being the molar extinction coefficient of uric acid, L being the light path of the cuvette, and C being the molar concentration of uric acid), the degradation concentration of uric acid and then the enzyme activity were calculated.

Definition of the activity of urate oxidase and PEGylated urate oxidase: at the optimal reaction temperature of 37°C and the optimal reaction pH of 9.5, the amount of enzyme required to convert 1 µmol uric acid into allantoin per minute being defined as one activity unit (U).

### 3. Stability effect of xanthine on urate oxidase from different sources

### (1) Comparison of the stability effect of xanthine on enzymes derived from Aspergillus flavus and mammals

In this example of the present disclosure, xanthine was added to the S1 and S2 samples at a molar ratio of 26 times (xanthine: urate oxidase). The samples were packaged and placed at 37°C for stability evaluation at days 0, 1.5, 6, and 16. The evaluation indicators include the content of high and low molecular proteins and enzyme specific activity. The quantitative results of small molecule content and enzyme activity in each group of samples are shown in Table 2.

**[Table 2] Stability effect of xanthine on urate oxidase from Aspergillus flavus and mammals**

| Storage conditions: 37°C+2°C | | Content of xanthine added µg/mL | High molecular weight % | Main peak% | Low molecular weight % | Enzyme specific activity U/mg |
|---|---|---|---|---|---|---|
| S1 | 0d | 0 | 0.3 | 98.8 | 1 | 15.3 |
| | 1.5d | | 0.2 | 94.2 | 5.6 | 12.6 |
| | 6d | | 7.3 | 75 | 17.7 | 8.5 |
| | 16d | | 17.2 | 31 | 51.7 | 3 |
| S1+26 times xanthine | 0d | 26.8 | 0.4 | 99.6 | / | 13.2 |
| | 1.5d | | 0.3 | 99.8 | / | 13.3 |
| | 6d | | 0.1 | 99.9 | / | 11.8 |
| | 16d | | 0.4 | 84.4 | 15.2 | 11.9 |
| S2 | 0d | 0 | 0.7 | 99 | 0.3 | 11.3 |
| | 1.5d | | 54.5 | 45.3 | 0.2 | 5.2 |
| | 6d | | 75.1 | 24.4 | 0.5 | 3.2 |
| | 16d | | 85.1 | 9.9 | 5 | 1.2 |
| S2+26 times xanthine | 0d | 60.7 | 2.6 | 97.4 | / | 10.8 |
| | 1.5d | | 3.9 | 95.8 | 0.4 | 10.4 |
| | 6d | | 7.6 | 91.3 | 1.1 | 10 |
| | 16d | | 21.3 | 75.5 | 3.1 | 8.5 |

By comparing the stability effects of xanthine on enzymes from different sources, it can be seen that xanthine can not only significantly improve the product stability of commercially available products and PHC, but also reduce the content of molecular protein. By comparing the detection results at 0d and on other days, it can be seen that the results of the detection method are stable.

### (2) Comparison of the stability effect of xanthine on enzymes from Micrococcus

**[Table 3] Stability effect of xanthine on urate oxidase from Micrococcus**

| Storage conditions: 2°C to 8°C | Total peak area | Main peak area | Main peak ratio% | High molecular weight% | Low molecular weight % | Content of xanthine added µg/mL | UV280 | Enzyme activity U/mg |
|---|---|---|---|---|---|---|---|---|
| Immediate detection after process preparation | 6914.90 | 3,986 | 57.65 | 40.01 | 2.34 | - | 1.1 | - |
| 15d | 4993.05 | 814 | 16.31 | 74.58 | 9.11 | - | 1.27 | 3.58 |
| 15d+26 times xanthine | 6779.70 | 1780 | 29.11 | 63.13 | 7.76 | 41.707 | 2.78 | 3.68 |

From the SEC results of the stability test at 2°C to 8°C in Table 3, it can be seen that the total peak area of the group with small molecules added on day 15 is larger and the main peak ratio thereof is higher. Xanthine can improve the stability of the enzyme derived from Micrococcus to a certain extent.

### Example 3: Preparation of urate oxidase or polyethylene glycol-modified urate oxidase containing xanthine during fermentation

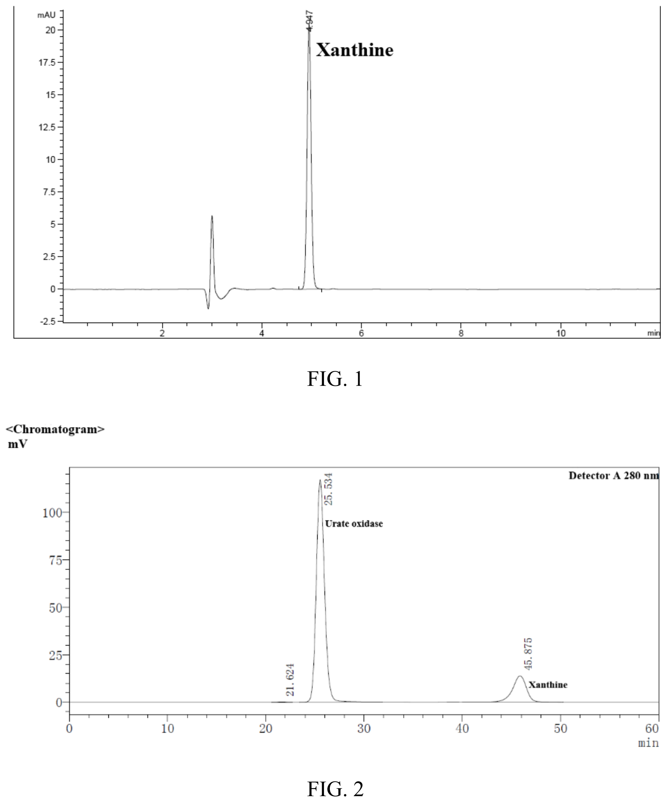

Urate oxidase was prepared by referring to the method described for enzyme S2 in Example 1, and the fermentation broth contained not less than 0.1 g/L of xanthine. The fermentation product was subjected to bacteria breaking and further purified by ion exchange or hydrophobic chromatography to prepare urate oxidase non-covalently bound to xanthine. RP-HPLC and SEC-HPLC detections showed that the expressed recombinant urate oxidase contained non-covalently bound xanthine. The RP-HPLC and SEC-HPLC detection graphs are shown in FIG. 1 and FIG. 2.

According to the method described in Example 1, the expressed recombinant urate oxidase was modified with polyethylene glycol.

### Example 4: Preparation of urate oxidase or polyethylene glycol-modified urate oxidase containing xanthine during purification

Urate oxidase was prepared by referring to the method described for enzyme S2 in Example 1. After breaking bacteria, ion exchange or hydrophobic chromatography, polyethylene glycol modification, and purification process, urate oxidase protein solution was obtained. In the urate oxidase protein solution containing a certain amount of xanthine, urate oxidase non-covalently bound to xanthine was prepared.

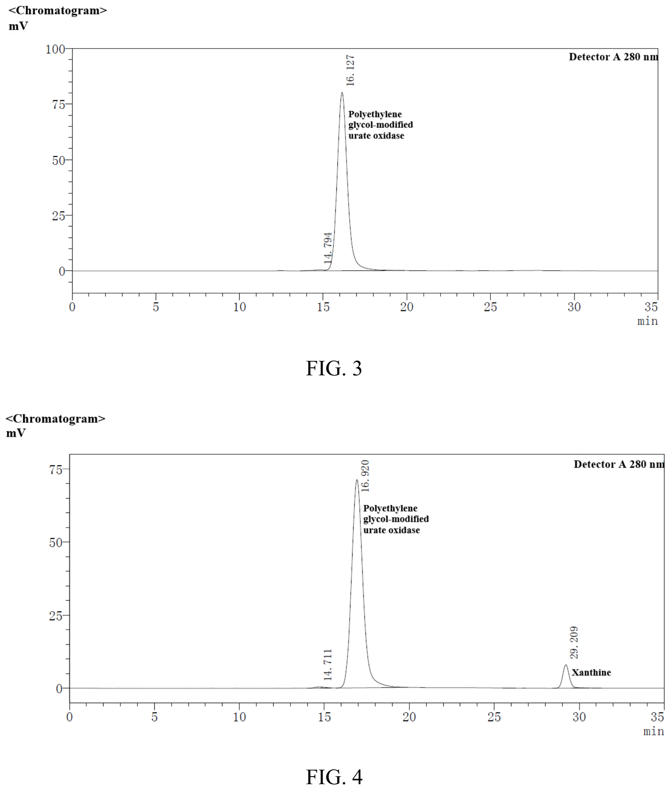

The polyethylene glycol-modified urate oxidase was prepared by referring to the method described for enzyme S2 in Example 1. After breaking bacteria, ion exchange or hydrophobic chromatography, polyethylene glycol modification, and purification process, PEGylated urate oxidase protein solution was obtained. A certain amount of xanthine was added before modifying urate oxidase protein with polyethylene glycol or added in the PEGylated urate oxidase protein solution, so that the PEGylated urate oxidase non-covalently bound to xanthine was prepared. The chromatograms detected by SEC-HPLC before and after the addition of xanthine are shown in FIG. 3 and FIG. 4.

### Example 5: Effects of different xanthine contents on the activity of urate oxidase and PEGylated urate oxidase

Referring to the detection method of enzyme activity in Example 2, the PEGylated urate oxidase was used as an example. The PEGylated urate oxidase was taken and dissolved in ultrapure water to prepare a solution. The PEGylated urate oxidase solution was mixed with different amounts of xanthine at a molar ratio of 0.05 mg/ml PEGylated urate oxidase to xanthine of 1:0, 1:3, 1:11, and 1:44, and then the activity of urate oxidase and polyethylene glycol-modified urate oxidase was determined according to the above-mentioned detection method. The measurement results are shown in Table 4.

**[Table 4] Effect of xanthine on the activity of PEGylated urate oxidase**

| Xanthine Concentration (µg/ml) | Blank control | | 0.075 | | 0.3 | | 1.2 | |
|---|---|---|---|---|---|---|---|---|
| A1 | 1.2887 | 1.2743 | 1.2809 | 1.2829 | 1.2725 | 1.2694 | 1.2717 | 1.2740 |
| A2 | 1.1994 | 1.1812 | 1.1893 | 1.1875 | 1.1792 | 1.1737 | 1.1780 | 1.1794 |
| A1-A2 | 0.0893 | 0.0931 | 0.0916 | 0.0954 | 0.0933 | 0.0957 | 0.0937 | 0.0946 |
| A1-A2 average | 0.0912 | | 0.0935 | | 0.0945 | | 0.0942 | |
| Enzyme specific activity (U/mg) | 10.70 | | 10.97 | | 11.09 | | 11.05 | |

As shown in Table 4 above, the addition of xanthine had no effect on the activity of PEGylated urate oxidase.

### Example 6: Effects of different xanthine contents on the stability of urate oxidase and PEGylated urate oxidase

The research results show that xanthine has good solubility under alkaline conditions. As the amount of xanthine added increases, the pH of the solution needs to be further increased to dissolve xanthine. However, high pH conditions will cause protein denaturation. After screening studies on the concentration of xanthine, the molar concentration ratio of xanthine to uricase was selected in the range of (1.6 to 26):1 to study the stability effect of xanthine on the uricase. Taking the modified PEGylated urate oxidase from mammals as an example, the final concentration of the PEGylated urate oxidase protein was 6 mg/ml, and PEGylated urate oxidase samples containing different concentrations of xanthine, 2.56 µg/ml, 42.24 µg/ml, and 168.96 µg/ml, were prepared and placed at 37°C for accelerated observation for 0, 1 month, and 2 months. The stability change trend of PEGylated urate oxidase tetramer was detected by the detection method of high and low molecular proteins of PEGylated urate oxidase. The test results are shown in Table 5:

**[Table 5] Effects of different xanthine contents on the stability of PEGylated urate oxidase (37°C)**

| Concentration of xanthine in PEGylated urate oxidase samples | Molar ratio of xanthine to PEGylated urate oxidase | SEC-HPLC low molecular protein | | |
|---|---|---|---|---|
| | | Day 0 | 1M | 2M |
| 0 µg/ml | 0:1 | Not detected | 11.2 | 19.1 |
| 0.56 µg/ml | 0.5:1 | Not detected | 11.2 | 19.1 |
| 2.56 µg/ml | 1.6:1 | Not detected | 11.2 | 18.4 |
| 42.24 µg/ml | 6.3:1 | Not detected | 5.7 | 11.2 |
| 168.96 µg/ml | 25.3:1 | Not detected | Not detected | 2.8 |

The results of the effects of different xanthine contents on the stability of PEGylated polyethylene glycol-modified urate oxidase showed that when the molar ratio of xanthine to PEGylated urate oxidase was in the range of (1.6:1) to (25.3:1), the stability of PEGylated urate oxidase tetramer tended to be more stable with the increase of concentration of xanthine. The results of Example 2 indicate that xanthine has a stability effect on urate oxidase from different sources within a 26 times molar ratio (xanthine: urate oxidase).

During the research process, the inventors found that when the content of xanthine reached 168.96 µg/ml, it was basically saturated. The situation where the molar ratio of xanthine to PEGylated urate oxidase was higher than 26:1 was studied. It was necessary to keep increasing the pH of the solution to increase the solubility of xanthine, since high pH conditions will cause urate oxidase protein denaturation and thus affect the effect of xanthine in improving stability. Therefore, at a concentration of 6 mg/ml PEGylated urate oxidase, the optimal molar ratio concentration range of xanthine to urate oxidase and PEGylated urate oxidase was selected in the range of (1-26): 1.

The researchers of the present disclosure found that the amount of xanthine added is related to the concentration of urate oxidase or PEGylated urate oxidase. As the concentration of urate oxidase or PEGylated urate oxidase in the urate oxidase solution increases, the amount of xanthine added can be reduced.

Based on the above research, the inventors studied the optimal amount of xanthine added in 1 mg/ml, 8 mg/ml, and 12 mg/ml urate oxidase solutions or PEGylated urate oxidase solutions with reference to the above-described method. The inventors found that when the final concentration of urate oxidase protein was 1 mg/ml, the molar ratio of xanthine to urate oxidase tetramer was (0.5 to 200): 1, preferably (1 to 156): 1, which had the best effect on improving the stability of urate oxidase or PEGylated urate oxidase; when the final concentration of urate oxidase was 8 mg/ml, the molar ratio of xanthine to urate oxidase tetramer was (0.5 to 25): 1, preferably (1 to 20): 1, which had the best effect on improving the stability of urate oxidase or PEGylated urate oxidase; when the final concentration of urate oxidase protein was 12 mg/ml, the molar ratio of xanthine to urate oxidase tetramer was (0.5 to 17): 1, preferably (1 to 13): 1, which had the best effect on improving the stability of urate oxidase or PEGylated urate oxidase.

In summary, within the range between 1 mg/ml and 12 mg/ml, the amount of xanthine added is in the range of (0.5 to 200): 1, preferably in the range of (1 to 156): 1 (molar ratio of xanthine to urate oxidase tetramer), which has the best effect on improving the stability of urate oxidase or PEGylated urate oxidase.

### Example 7: Effects of xanthine on the pharmacokinetics and pharmacodynamics of PEGylated urate oxidase injection in rats

Taking the modified PEGylated urate oxidase from mammals as an example, the pharmacokinetic and pharmacodynamic indicators of xanthine-containing and xanthine-free PEGylated urate oxidase in rats were evaluated in vivo.

The principle of pharmacokinetic (PK) assay of PEGylated urate oxidase was as follows. The concentration of PEGylated urate oxidase injection in SD rat serum samples was determined by a methodologically validated analytical method (fluorescence method) that the uricase catalyzed the conversion of uric acid into allantoin, H₂O₂, and CO₂. In the presence of horseradish peroxidase (HRP), H₂O₂ reacted with Amplex Red Reagent (10-acetyl-3,7-dihydroxyphenazine) in a 1:1 ratio to generate a red fluorescent oxidation product resorufin. The amount of PEGylated uricase injection was correlated with the depth of color produced by the final reaction. When fluorescence detection was performed, the excitation light was set to 485 nm, and the emission light was set to 630 nm.

The principle of detection of uric acid in serum (PD) was as follows. The concentration of uric acid in SD rat serum samples was directly detected by a biochemical analyzer (Enzymatic Colorimetric Test method). The uric acid in the sample generated allantoin and hydrogen peroxide (H₂O₂) under the action of uricase. H₂O₂ oxidized 4-aminoaminopyrine (4-AAP) under the action of peroxidase (POD) to generate red quinone diimine. The absorbance change was measured at 520 nm and was proportional to the concentration of uric acid in the sample.

The reaction formula was:

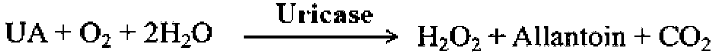

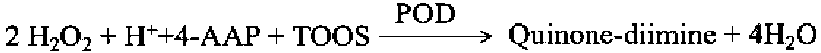

Test samples: xanthine-free PEGylated urate oxidase, code: PEG-Uricase (-X); xanthine-containing PEGylated urate oxidase, code: PEG-Uricase (+X), each 1 mg of PEGylated urate oxidase protein containing 1.5 µg of xanthine.

Animal grouping: twelve SD rats were taken and randomly divided into two groups, with 6 rats in each group (half male and half female). The administration dose was 1 mg/kg according to the content of urate oxidase protein, using a single intravenous administration, namely the PEG-Uricase (-X) -i.v. group and the PEG-Uricase (+X) -i.v. group.

Blood collection time points: blood was collected before administration, and 1 day, 3 days, 5 days, 7 days, 9 days, 11 days, and 13 days after administration, to measure blood drug concentration and uric acid level in serum.

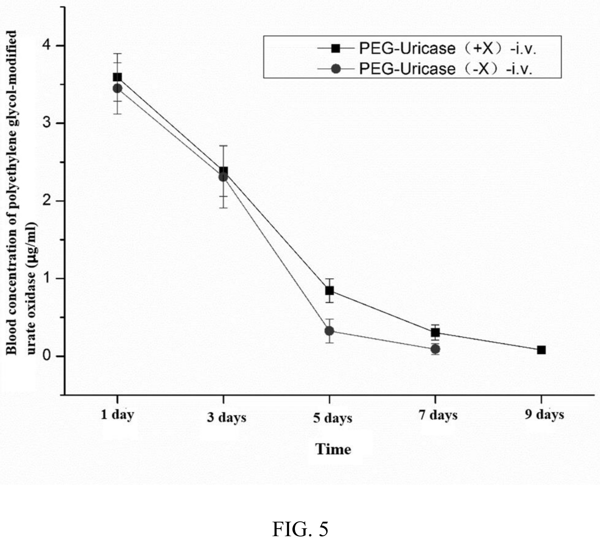

The statistics of blood drug concentration measurement are shown in Table 6 and the curve graph of changes in blood concentration is shown in FIG. 5. It can be seen that on the 5th day, the concentration of xanthine-containing PEGylated urate oxidase in the blood was much greater than the concentration of xanthine-free PEGylated urate oxidase, and on the 9th day, the concentration of xanthine-free PEGylated urate oxidase in the blood was completely below the detection limit, while the average concentration of xanthine-containing PEGylated urate oxidase in the blood was 0.081 µg/ml. This shows that the xanthine added can improve the stability of PEGylated urate oxidase in the blood and slow down the clearance of PEGylated urate oxidase by the system.

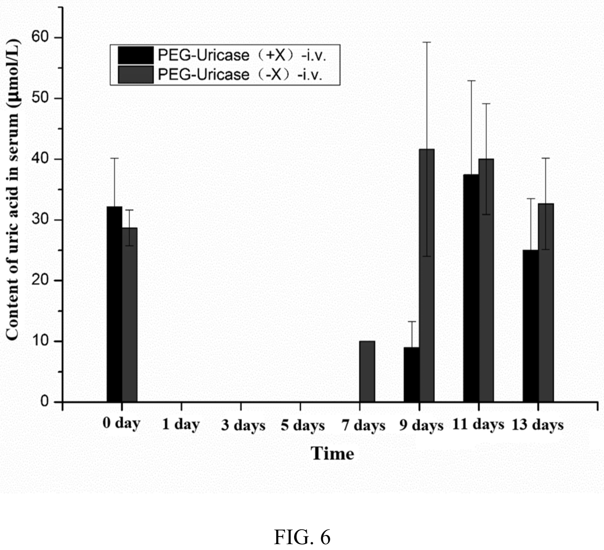

The statistics of the content of uric acid in serum are shown in Table 7 and the graph of changes in uric acid levels in serum is shown in FIG. 6. It can be seen that within the first 5 days, the contents of uric acid of both xanthine-containing PEGylated urate oxidase and xanthine-free PEGylated urate oxidase in serum were below the detection limit. On the 7th day, the content of uric acid of xanthine-free PEGylated urate oxidase in serum was above the detection limit, while the content of uric acid of xanthine-containing PEGylated urate oxidase in serum was still below the detection limit. On the 9th day, the content of uric acid of xanthine-free PEGylated urate oxidase in serum was much higher than that of xanthine-containing PEGylated urate oxidase in serum, and in the following days, the content of uric acid of xanthine-free PEGylated urate oxidase was always higher than that of xanthine-containing PEGylated urate oxidase. This shows that the stability of xanthine-containing PEGylated urate oxidase in serum is significantly higher than that of xanthine-free PEGylated urate oxidase, and it can play the role of inhibiting the formation of uric acid in serum and reducing the content of uric acid in serum for a longer time.

**[Table 6] Statistical table of blood drug concentration measurement**

| Group | Animal No. | Blood drug concentration (µg/ml) | | | | | | | |
|---|---|---|---|---|---|---|---|---|---|
| | | Day 0 | Day 1 | Day 3 | Day 5 | Day 7 | Day 9 | Day 11 | Day 13 |
| PEG-Uricase (+X) -i.v. | 1 | N.D. | 3.688 | 2.462 | 0.892 | 0.152 | N.D. | N.D. | N.D. |
| | 2 | N.D. | 3.853 | 2.482 | 0.863 | 0.275 | 0.089 | N.D. | N.D. |
| | 3 | N.D. | 3.359 | 2.867 | 0.711 | 0.324 | N.D. | N.D. | N.D. |
| | 4 | N.D. | 3.387 | 2.279 | 0.624 | 0.371 | 0.072 | N.D. | N.D. |
| | 5 | N.D. | 4.019 | 2.369 | 0.964 | 0.403 | N.D. | N.D. | N.D. |
| | 6 | N.D. | 3.247 | 1.862 | 1.02 | N.D. | N.D. | N.D. | N.D. |
| | Mean | N.D. | 3.592 | 2.387 | 0.846 | 0.305 | 0.081 | N.D. | N.D. |
| | SD | / | 0.308 | 0.326 | 0.151 | 0.098 | 0.012 | / | / |
| PEG-Uricase (-X) -i.v. | 1 | N.D. | 3.293 | 2.491 | 0.212 | N.D. | N.D. | N.D. | N.D. |
| | 2 | N.D. | 3.547 | 2.114 | 0.201 | 0.209 | N.D. | N.D. | N.D. |
| | 3 | N.D. | 3.216 | 2.259 | 0.613 | 0.094 | N.D. | N.D. | N.D. |
| | 4 | N.D. | 3.888 | 2.572 | 0.226 | 0.031 | N.D. | N.D. | N.D. |
| | 5 | N.D. | 3.018 | 1.653 | 0.345 | 0.047 | N.D. | N.D. | N.D. |
| | 6 | N.D. | 3.737 | 2.772 | 0.352 | 0.075 | N.D. | N.D. | N.D. |
| | Mean | N.D. | 3.450 | 2.310 | 0.325 | 0.091 | N.D. | N.D. | N.D. |
| | SD | / | 0.332 | 0.397 | 0.156 | 0.070 | / | / | / |

| | | | | | | | | | |
|---|---|---|---|---|---|---|---|---|---|
| **[00160]** * Note: N.D. means that the content was below the detection limit, the same below. | | | | | | | | | |

**[Table 7] Statistics of content of uric acid in serum**

| Group | Animal No. | Content of uric acid (µmol/L) | | | | | | | |
|---|---|---|---|---|---|---|---|---|---|
| | | Day 0 | Day 1 | Day 3 | Day 5 | Day 7 | Day 9 | Day 11 | Day 13 |
| PEG-Uricase (+X) -i.v. | 1 | 25 | N.D. | N.D. | N.D. | N.D. | 6 | 16 | 25 |
| | 2 | 44 | N.D. | N.D. | N.D. | N.D. | N.D. | N.D. | 13 |
| | 3 | 29 | N.D. | N.D. | N.D. | N.D. | 15 | 46 | 24 |
| | 4 | 40 | N.D. | N.D. | N.D. | N.D. | 9 | 27 | 38 |
| | 5 | 25 | N.D. | N.D. | N.D. | N.D. | 6 | 54 | 20 |
| | 6 | 30 | N.D. | N.D. | N.D. | N.D. | N.D. | 44 | 30 |
| | Mean | 32.17 | N.D. | N.D. | N.D. | N.D. | 9 | 37.4 | 25 |
| | SD | 7.99 | / | / | / | / | 4.24 | 15.49 | 8.53 |
| PEG-Uricase (-X) -i.v. | 1 | 29 | N.D. | N.D. | N.D. | 10 | 41 | 32 | 41 |
| | 2 | 31 | N.D. | N.D. | N.D. | N.D. | 57 | 36 | 27 |
| | 3 | 27 | N.D. | N.D. | N.D. | N.D. | 52 | 53 | 35 |
| | 4 | 25 | N.D. | N.D. | N.D. | N.D. | 12 | 41 | 21 |
| | 5 | 27 | N.D. | N.D. | N.D. | N.D. | 46 | 30 | 33 |
| | 6 | 33 | N.D. | N.D. | N.D. | N.D. | N.D. | 48 | 39 |
| | Mean | 28.67 | N.D. | N.D. | N.D. | 10 | 41.6 | 40 | 32.67 |
| | SD | 2.94 | / | / | / | / | 17.62 | 9.10 | 7.53 |

In the specification, the description of the reference terms such as "an embodiment", "some embodiments", "example", "specific example", or "some examples" means that the specific features, structures, materials, or characteristics described with reference to the embodiment or example are included in at least an embodiment or example of the present disclosure. In this specification, exemplary descriptions of the foregoing terms do not necessarily refer to the same embodiment or example. Moreover, the specific features, structures, materials, or characteristics described may be combined in any one or more embodiments or examples in a suitable manner. Furthermore, those skilled in the art may combine different embodiments or examples and features of different embodiments or examples described in this specification, unless they are contradictory to each other.

Although embodiments of the present disclosure are illustrated and described above, it can be understood that the above embodiments are illustrative and should not be construed as limitations of the present disclosure. Those skilled in the art can make changes, modifications, substitutions, and variations to the above embodiments within the scope of the present disclosure.

## Claims

1. A urate oxidase composition, comprising:
an active ingredient, comprising urate oxidase; and
a stabilizer, comprising xanthine.

2. The urate oxidase composition according to claim 1, wherein a molar ratio of xanthine to urate oxidase tetramer is not less than 0.5:1.

3. The urate oxidase composition according to claim 1, wherein the urate oxidase is extracted natural urate oxidase, expressed recombinant urate oxidase, or modified urate oxidase.

4. The urate oxidase composition according to claim 3, wherein:
the extracted natural urate oxidase is derived from *Aspergillus flavus,* wild-type Micrococcus, or mammals; and
the modified urate oxidase is selected from the group consisting of polyethylene glycol-modified urate oxidase, polysaccharide side chain-modified urate oxidase, polyoligopeptide-modified urate oxidase, and a combination thereof.

5. The urate oxidase composition according to claim 1, wherein the urate oxidase is bound to xanthine through a non-covalent bond.

6. The urate oxidase composition according to claim 5, wherein the urate oxidase is bound to xanthine through hydrogen bonding, salt bonding, hydrophobic interaction, or van der Waals force.

7. The urate oxidase composition according to claim 1, wherein:
a concentration of the urate oxidase in the composition ranges from 1 mg/ml to 12 mg/ml;
and
a molar ratio of xanthine to urate oxidase tetramer is (0.5 to 200): 1, and preferably (1 to 156): 1.

8. The urate oxidase composition according to claim 1, wherein:
a concentration of the urate oxidase in the composition is 1 mg/ml; and
a molar ratio of xanthine to urate oxidase tetramer is (0.5 to 200): 1, and preferably (1 to 156): 1.

9. The urate oxidase composition according to claim 1, wherein:
a concentration of the urate oxidase in the composition is 6 mg/ml; and
a molar ratio of xanthine to urate oxidase tetramer is (0.5 to 34): 1, and preferably (1 to 26): 1.

10. The urate oxidase composition according to claim 1, wherein:
a concentration of the urate oxidase in the composition is 8 mg/ml; and
a molar ratio of xanthine to urate oxidase tetramer is (0.5 to 25): 1, and preferably (1 to 20): 1.

11. The urate oxidase composition according to claim 1, wherein:
a concentration of the urate oxidase in the composition is 12 mg/ml; and
a molar ratio of xanthine to urate oxidase tetramer is (0.5 to 17): 1, and preferably (1 to 13): 1.

12. The urate oxidase composition according to claim 1, further comprising a pharmaceutically acceptable excipient or carrier, wherein:
the excipient or carrier is selected from the group consisting of hydroxypropyl methylcellulose, hydroxyethyl cellulose, hydroxypropyl cellulose, mannitol, sucrose, fructose, methyl cellulose, vinyl acetate copolymer, chitosan, sodium alginate, xanthan gum, sodium carboxymethyl cellulose, polyethylene oxide, methacrylic acid copolymer, maleic anhydride-methyl vinyl ether copolymer, carbomer, polyvinyl pyrrolidone, ethyl cellulose, cellulose acetate, methacrylate, polyoxyethylene, polyvinyl alcohol, glyceryl behenate, glycerol monostearate, cellulose acetate, cellulose acetate phthalate, ethyl cellulose, PLA, PLGA, polyethylene glycol, and combinations thereof.

13. The urate oxidase composition according to claim 12, wherein the composition is an injection.

14. Use of xanthine in improving the stability of urate oxidase.

15. A method for improving the stability of urate oxidase, comprising contacting the urate oxidase with xanthine.

16. A method for preparing urate oxidase, comprising:
contacting urate oxidase with xanthine.

17. The method according to claim 15 or 16, wherein the urate oxidase is extracted natural urate oxidase, expressed recombinant urate oxidase, or modified urate oxidase.

18. The method according to claim 17, wherein:
the extracted natural urate oxidase is derived from *Aspergillus flavus,* wild-type Micrococcus, or mammals; and
the modified urate oxidase is selected from the group consisting of polyethylene glycol-modified urate oxidase, polysaccharide side chain-modified urate oxidase, polyoligopeptide-modified urate oxidase, and a combination thereof.

19. The method according to claim 15 or 16, wherein said contacting urate oxidase with xanthine is performed by:
fermenting engineered bacteria carrying a nucleic acid molecule expressing the urate oxidase in a xanthine-containing fermentation broth, to obtain the urate oxidase.

20. The method according to claim 19, wherein an amount of xanthine in the xanthine-containing fermentation broth is not less than 0.1 g/L.

21. The method according to claim 19, further comprising:
performing a polyethylene glycol modification and purification treatment on the urate oxidase, to obtain polyethylene glycol-modified urate oxidase.

22. The method according to any one of claims 15 to 18, wherein said contacting urate oxidase with xanthine is performed by:
contacting polyethylene glycol-modified urate oxidase with xanthine.

23. A urate oxidase, obtained by the method according to any one of claims 16 to 22.

24. Use of the urate oxidase composition according to any one of claims 1 to 13 or the urate oxidase according to claim 23 in the manufacture of a medicament for preventing or treating a hyperuricemia-related disease.

25. The use according to claim 24, wherein the hyperuricemia-related disease comprises chronic hyperuricemia, gout, kidney disease, hyperuricemia arthritis, kidney stones, gouty nodules, hypertension, diabetes, hypertriglyceridemia, metabolic syndrome, coronary heart disease, atherosclerosis, or hyperuricemia caused by cancer chemotherapy.

26. Use of the urate oxidase composition according to any one of claims 1 to 13 or the urate oxidase according to claim 23 in reducing a uric acid level in a biological fluid, wherein the biological fluid is urine or blood.

27. A medicament, comprising:
the urate oxidase composition according to any one of claims 1 to 13 or the urate oxidase according to claim 23; and
a pharmaceutically acceptable carrier and/or excipient.

28. Use of the urate oxidase composition according to any one of claims 1 to 13 or the urate oxidase according to claim 23 in preventing or treating a hyperuricemia-related disease.

29. A method for preventing or treating a hyperuricemia-related disease, comprising:
administering to a subject a therapeutically effective amount of the urate oxidase composition according to any one of claims 1 to 13, the urate oxidase according to claim 23, or the medicament according to claim 27.

30. The use according to claim 28 or the method according to claim 29, wherein the hyperuricemia-related disease comprises chronic hyperuricemia, gout, kidney disease, hyperuricemia arthritis, kidney stones, gouty nodules, hypertension, diabetes, hypertriglyceridemia, metabolic syndrome, coronary heart disease, atherosclerosis, or hyperuricemia caused by cancer chemotherapy.
